# EUROPÄISCHE PATENTSCHRIFT

(11) **EP 4 455 648 B1**
(45) Veröffentlichungstag und Bekanntmachung des Hinweises auf die Patenterteilung: **21.05.2025**
(21) Anmeldenummer: 24171590.3
(22) Anmeldetag: 22.04.2024
(51) Int. Cl.: G01N 22/04

(54) **FEUCHTESENSOR, VERFAHREN ZUR FEUCHTEMESSUNG UND VERFAHREN ZUR HERSTELLUNG EINES ZUR FEUCHTEMESSUNG EINGERICHTETEN BAUTEILS**
MOISTURE SENSOR, METHOD FOR MEASURING MOISTURE AND METHOD FOR PRODUCING A COMPONENT DESIGNED FOR MEASURING MOISTURE
CAPTEUR D'HUMIDITÉ, PROCÉDÉ DE MESURE D'HUMIDITÉ ET PROCÉDÉ DE FABRICATION D'UN COMPOSANT CONÇU POUR LA MESURE D'HUMIDITÉ

(30) Priorität: 26.04.2023 DE 102023110648
(43) Veröffentlichungstag der Anmeldung: 30.10.2024
(73) Patentinhaber: Freistaat Thüringen (Materialforschungs- und -prüfanstalt an der Bauhaus-Universität Weimar), 99423 Weimar (DE)
(72) Erfinder: HELBIG, Dr. Stefan, 99428 Weimar (DE); WAGNER, Dr. Ralf, 99096 Erfurt (DE); BONITZ, Frank, 99423 Weimar (DE); KUHNE, Dr. Michael, 99423 Weimar (DE); MÜLLER, Bernd, 99098 Erfurt (DE); ULANOV, Alexander, 99425 Weimar (DE)
(74) Vertreter: Liedtke & Partner Patentanwälte

(56) Entgegenhaltungen:
- DE-A1- 102017 111 962
- DE-B3- 102005 013 647
- US-A- 4 103 224
- SIDEN JOHAN ET AL: "Microstrip antennas for remote moisture sensing using passive RFID", 2009 ASIA PACIFIC MICROWAVE CONFERENCE, 1 December 2009 (2009-12-01), pages 2375 - 2378, XP093205143, Retrieved from the Internet <URL:https://ieeexplore.ieee.org/stampPDF/getPDF.jsp?tp=&arnumber=5385463&ref=aHR0cHM6Ly9pZWVleHBsb3JlLmllZWUub3JnL2Fic3RyYWN0L2RvY3VtZW50LzUzODU0NjM=> DOI: 10.1109/APMC.2009.5385463

## Beschreibung

Die Erfindung betrifft nach einem ersten Aspekt einen zur Feuchtemessung einer Umgebung eingerichteten Feuchtesensor umfassend ein elektromagnetisch abschirmendes Sensorgehäuse und einen darin angeordneten Streifenleiter sowie mindestens ein Koaxialkabel mit je einem mit dem Streifenleiter elektrisch verbundenen Innenleiter und einem mit dem Sensorgehäuse elektrisch verbundenen Außenleiter. Ferner betrifft die Erfindung nach einem zweiten Aspekt ein Verfahren zur Feuchtemessung mit einem solchen Feuchtesensor. Zudem betrifft die Erfindung nach einem dritten Aspekt ein Verfahren zur Herstellung eines Bauteils mit einem darin eingebrachten Feuchtesensor.

Aus dem Stand der Technik sind als Ausgleichsfeuchtemessung bezeichnete Verfahren zum Nachweis einer Feuchtebelastung in Bauteilen bekannt, bei denen eine relative Feuchte als Ausgleichsfeuchte zu einem angrenzenden feuchten Feststoff bestimmt wird. Insbesondere sind als hygrometrische Verfahren bezeichnete Verfahren zur Messung von Feuchtebelastungen im hygroskopischen Bereich bekannt, beispielsweise aus Kupfer, K.: Materialfeuchtemessung: Grundlagen, Messverfahren, Applikationen, Normen. Renningen-Malmsheim, expert-Verlag, 1997.

Das Dokument DE 10 2005 013 647 B3 betrifft ein Verfahren zur Messung der Materialfeuchte eines Messgutes mithilfe eines Mikrowellenresonators, bei dem das Messgut in den Wirkbereich des Resonators eingebracht wird und die Materialfeuchte aus der Änderung der Güte und der Resonatorfrequenz des Resonators bestimmt wird, wobei die in den Resonator eingespeiste Frequenz variiert und dabei mindestens die Resonanzkurve des Resonators überstrichen wird, wobei die Güte und die Resonanzfrequenz mittels zweier elektrischer Leiter gemessen wird, von denen sich der eine innerhalb und der andere außerhalb des Wirkungsbereiches des Resonators befindet und zur Bestimmung der Resonatorgüte die frequenzabhängige Phasendifferenz und zur Bestimmung der Resonanzfrequenz das frequenzabhängige Dämpfungsverhältnis der zwei Leiter gemessen wird.

Das Dokument DE 10 2017 111 962 A1 beschreibt ein Verfahren zur Bestimmung der absoluten Bauteilfeuchte. Das Verfahren umfasst die folgenden Schritte: Einbringen oder Aufbringen einer Sensorvorrichtung mit einem Feuchte-Sensor in oder an ein Bauteil; zerstörungsfreies Bestimmen der Luftfeuchte mit der Sensorvorrichtung an einer Position im oder am Bauteil; und Bestimmen der absoluten Bauteilfeuchte aus der bestimmten Luftfeuchte. Weiterhin werden ein System zur zerstörungsfreien Bestimmung der absoluten Bauteilfeuchte und die Verwendung eines Luftfeuchte-Sensors zur zerstörungsfreien Bestimmung der absoluten Bauteilfeuchte an einer Position in oder an einem Bauteil beschrieben.

Das Dokument DE 33 39 602 A1 beschreibt einen Feuchtigkeitsfühler mit einer Mikrowellenübertragungsleitung, deren Dielektrikum mindestens teilweise von dem hinsichtlich seines Feuchtigkeitsgehalts zu messenden Material gebildet wird und wobei als Maß für den Feuchtigkeitsgehalt die Dämpfung der Mikrowellenenergie auf der Übertragungsleitung gemessen wird. Zur Feuchtigkeitsmessung bei anisotropen Materialien verläuft die Übertragungsleitung längs eines nicht geradlinigen Pfades mit etwa gleichen Längenabschnitten, die in einer Vielzahl unterschiedlicher Richtungen verlaufen, die alle mit gleichen Winkelabständen über einem Bogenquadranten angeordnet sind. Dabei kann der Leitungspfad als mäanderförmige Folge von Kreisbogenquadranten und die Leitung zweckmäßig als Schlitzleitung ausgebildet sein.

Das Dokument DE 33 17 200 A1 beschreibt ein Verfahren und eine Vorrichtung zur Messung der Feuchte von Schüttgütern, wie feuchte feinkörnige Kohle, Erze, Sande oder dergleichen, mit dem Kennzeichen, dass eine Leitung mit MikrowellenGenerator und Antenne durch Anpassung des Wellenwiderstandes der Leitung an den Wellenwiderstand der Umgebung bestimmter Feuchte frei von Wellenreflexionen gemacht wird, dass die Antenne in das zu messende feuchte Schüttgut eingeführt wird und dass bei einer Änderung der Feuchte der Antennenumgebung ein Teil der von der Antenne abgestrahlten Energie in die Leitung reflektiert und von einem Detektor gemessen wird.

Der Erfindung liegt gemäß ihrem ersten Aspekt die Aufgabe zu Grunde, einen verbesserten Feuchtesensor zur Feuchtemessung einer Umgebung anzugeben. Diese Aufgabe wird mit einem Feuchtesensor mit den Merkmalen des Anspruchs 1 gelöst.

Gemäß ihrem zweiten Aspekt liegt der Erfindung die Aufgabe zu Grunde, ein verbessertes Verfahren zur Feuchtemessung anzugeben. Diese Aufgabe wird mit einem Verfahren mit den Merkmalen des Anspruchs 7 gelöst.

Gemäß ihrem dritten Aspekt liegt der Erfindung die Aufgabe zu Grunde, ein verbessertes Verfahren zur Herstellung eines Bauteils anzugeben. Diese Aufgabe wird mit einem Verfahren mit den Merkmalen des Anspruchs 12 gelöst.

Vorteilhafte Ausgestaltungen der Erfindung sind Gegenstand der Unteransprüche.

Gemäß dem ersten Aspekt der Erfindung umfasst ein Feuchtesensor ein elektromagnetisch abschirmendes Sensorgehäuse mit einem darin angeordneten Streifenleiter sowie mit mindestens einem Koaxialkabel, das zur Leitung von Mikrowellen mit einer Frequenz innerhalb eines vorbestimmten Frequenzbands eingerichtet ist. Das Frequenzband überdeckt mindestens einen Teilbereich des Mikrowellenbereichs von zwischen 10 Megahertz (MHz) und 300 Gigahertz (GHz).

Das mindestens eine Koaxialkabel weist einen Innenleiter und einen Außenleiter auf, wobei der Innenleiter mit dem Streifenleiter elektrisch verbunden ist und der Außenleiter mit dem Sensorgehäuse elektrisch verbunden ist.

Das Sensorgehäuse ist als Hohlraumresonator mit einer Resonanzfrequenz eingerichtet, die innerhalb des vorbestimmten Frequenzbands liegt, und ist zudem für Frequenzen in diesem Frequenzband elektromagnetisch abschirmend ausgebildet. Hierzu kann das Sensorgehäuse aus elektrisch leitendem Material hergestellt und/oder mit einem elektrisch leitenden Material beschichtet sein.

Das Sensorgehäuse weist Öffnungen auf, die für einen hygrischen Kontakt vorgesehen und eingerichtet sind, das heißt: für eine Durchlässigkeit von Wasser und/oder Wasserdampf, welche für den Feuchteausgleich zwischen dem Inneren des Sensorgehäuses und dessen Umgebung ausreicht. In den Innenraum des Sensorgehäuses ist ein feuchtespeicherndes Material eingebracht. Das feuchtespeichernde Material weist bevorzugt eine ähnliche Feuchtespeicherfunktion (das heißt: eine ähnliche Sorptionsisotherme) auf wie das zu vermessende Material in der Umgebung des Feuchtesensors (Messobjekt). In einer speziellen, nachfolgend noch genauer beschriebenen Ausführungsform ist das feuchtespeichernde Material im Innenraum des Sensorgehäuses dasselbe wie das Material in der Umgebung des Feuchtesensors.

Über den durch die Öffnungen vermittelten hygrischen Kontakt wird Feuchte (Wasser oder Wasserdampf) aus der Umgebung des Sensorgehäuses in dessen Inneres transportiert (beispielsweise durch kapillares Saugen oder durch Diffusion), bis ein Feuchteausgleich zwischen dem Innenraum und der Umgebung des Sensorgehäuses hergestellt ist.

Indem das Sensorgehäuse (durch Herstellung aus bzw. Beschichtung mit elektrisch leitfähigen Materialien) elektromagnetisch abschirmend ausgestaltet ist, können über das mindestens eine Koaxialkabel elektromagnetische Wellen im Mikrowellenbereich eingekoppelt werden, welche sich praktisch nur im Inneren des Sensorgehäuses, nicht jedoch in dessen Umgebung ausbreiten.

Indem das Sensorgehäuse als Hohlraumresonator ausgebildet ist, wird dessen Resonanzfrequenz von den dielektrischen Eigenschaften im Inneren des Sensorgehäuses beeinflusst, welche wiederum von der Feuchtigkeit im Gehäuseinneren und somit, über den Feuchteausgleich, auch von der Feuchtigkeit in der Umgebung abhängen.

Der erfindungsgemäße Feuchtesensor ermöglicht somit über die Bestimmung der Resonanzfrequenz eine Ermittlung der Feuchte in der Umgebung des Sensorgehäuses und ist besonders einfach, mit geringem Materialaufwand und kostensparend herstellbar. Insbesondere kann ein derartiger Sensor auch für den dauerhaften Verbleib in einem Bauteil, beispielsweise in einem aus Beton oder Estrich gegossenen Bauwerks-Bauteil, vorgesehen werden. Zudem kann durch geeignete Wahl des in dem Sensorgehäuse eingebrachten feuchtespeichernden Materials, insbesondere unter Berücksichtigung von dessen Feuchtespeicherfunktion, ein Messbereich von besonders hoher Empfindlichkeit für die Feuchte des den Feuchtesensor umgebenden Materials sehr leicht eingestellt werden.

Bei einer Ausführungsform ist ein erstes Koaxialkabel in das Sensorgehäuse geführt und mit seinem Innenleiter mit einem ersten Ende der Streifenleitung elektrisch verbunden. Ein zweites Koaxialkabel ist ebenfalls in das Sensorgehäuse geführt und ist mit seinem Innenleiter mit einem dem ersten Ende gegenüberliegenden zweiten Ende der Streifenleitung elektrisch verbunden. Das zweite Koaxialkabel kann dem ersten Koaxialkabel gegenüberliegend in das Sensorgehäuse geführt sein.

Diese Ausführungsform ermöglicht sowohl transmissive als auch reflektive Messungen des Durchgangs (das heißt: der Weiterleitung) beziehungsweise der Reflexion von Mikrowellen. Sie ist dadurch besonders vielseitig einsetzbar und ermöglicht eine besonders einfache und genaue Bestimmung der Feuchte.

Bei einer Ausführungsform ist das Sensorgehäuse als Rechteck-Hohlleiter ausgebildet und weist zwei kongruente Grundflächen auf, die einem Abstand parallel gegenüberliegend angeordnet sind, der kleiner als die kleinste Abmessung dieser Grundflächen ist. Die Grundflächen sind über Seitenflächen des Rechteck-Hohlleiters miteinander verbunden. In mindestens eine der Grundflächen sind Öffnungen zum Feuchteausgleich zwischen dem Inneren des Sensorgehäuses und dessen Umgebung eingebracht. Das mindestens eine Koaxialkabel ist durch je eine der Seitenflächen in das Sensorgehäuse geführt, wobei der Innenleiter mit dem Streifenleiter und der Außenleiter mit dem Sensorgehäuse verbunden sind.

Diese Ausführungsform weist den Vorteil einer besonders einfachen Konstruktion auf.

Bevorzugt ist bei einer Ausführungsform des Feuchtesensors mit einem als Rechteck-Hohlleiter ausgebildeten Sensorgehäuse ein erstes Koaxialkabel zur Einkopplung von Mikrowellen bestimmt und durch eine erste Seitenfläche in das Sensorgehäuse geführt und mit seinem Innenleiter mit einem ersten Ende der Streifenleitung elektrisch verbunden. Ein zweites Koaxialkabel ist zur Auskopplung oder Weiterleitung von eingekoppelten Mikrowellen bestimmt und durch eine der ersten Seitenfläche gegenüberliegende zweite Seitenfläche in das Sensorgehäuse geführt und mit seinem Innenleiter mit einem dem ersten Ende gegenüberliegenden zweiten Ende der Streifenleitung elektrisch verbunden. Alternativ kann das zweite Koaxialkabel auch an einer anderen Seitenfläche in das Sensorgehäuse geführt sein.

Unabhängig von der Anzahl der in das Sensorgehäuse geführten Koaxialkabel ist entweder eine einseitige oder zweiseitige Perforation des Sensorgehäuses für einen Feuchteausgleich mit der Umgebung möglich.

Neben einer Ausbildung des Sensorgehäuses als Rechteck-Hohlleiter sind auch Ausführungsformen möglich, bei denen das Sensorgehäuse als Hohlleiter in anderer Geometrie gestaltet ist. Beispielsweise kann das Sensorgehäuse als Hohlzylinder in der Art eines Rohrabschnitts ausgebildet sein, dessen Mantelfläche perforiert ist. Im Inneren eines solchen Hohlzylinders kann als Streifenleiter beispielsweise ein Stab aus leitfähigem Material oder eine Leiterplatte mit mindestens einem darauf aufgebrachten Leiterzug angeordnet sein. Bevorzugt ist der Streifenleiter mittig (im Zentrum entlang der Zylinderachse des Hohlzylinders verlaufend) angeordnet.

Auch Ausführungsformen in derartigen (von einem Rechteck-Hohlleiter abweichenden) Sensorgehäuseformen können mit einem Koaxialkabel oder mit zwei Koaxialkabeln versehen sein, um eine reflektive beziehungsweise eine reflektive und/oder transmissive Messung zu ermöglichen.

Bei einer Ausführungsform ist die Streifenleitung als Kupferplatte ausgeführt. Diese Ausführungsform kann besonders einfach hergestellt werden.

Alternativ ist die Streifenleitung als kupferbeschichtete Leiterplatte, bevorzugt mit einer Leitungsführung in der Art einer Einstreifenleitung, einer Mehrstreifenleitung oder einer daraus gebildeten Leiterstruktur ausgeführt. Eine solche Leiterstruktur kann beispielsweise als mäanderförmig auf einer Leiterplatte verlaufender Leiterzug (Einstreifenleitung) oder mäanderförmig parallel zueinander verlaufende Mehrzahl von Leiterzügen (Mehrstreifenleitung) mit einem ersten und einem zweiten Ende ausgebildet sein.

Alternativ können Leiterstrukturen auch als Interdigitalstrukturen ausgebildet werden.

Mit derartigen Streifenleitungen versehene Sensorgehäuse weisen ein besonders gutes Resonanzverhalten, das heißt: eine besonders einfach und genau zu bestimmende Resonanzfrequenz auf.

Bei einer Ausführungsform ist das in den Innenraum des Sensorgehäuses eingebrachte feuchtespeichernde Material abhängig von dem Material gewählt, in welches der Feuchtesensor eingebracht werden soll und dessen Feuchte damit gemessen werden soll. Es soll eine Feuchtespeicherfunktion besitzen, die der Feuchtespeicherfunktion des Messobjektes oder zumindest ähnlich ist.

Unter einer zur Feuchtespeicherfunktion des Messobjekts ähnlichen Feuchtespeicherfunktion soll hier und im Folgenden eine Feuchtespeicherfunktion verstanden werden, gemäß der das Material im Inneren des Sensorgehäuses und das Material in der Umgebung bei der gleichen relativen Luftfeuchte (beziehungsweise Porenluftfeuchte) jeweils gleich viel volumenbezogene Feuchte aufnehmen können.

Insbesondere bezieht sich diese Gleichheit auf den für den Sensor angestrebten Messbereich (das heißt: den Bereich der zugehörigen Porenluftfeuchten). Eine Ähnlichkeit der Feuchtespeicherfunktionen ist auch dann vorhanden, wenn die funktionalen Verläufe der Feuchtespeicherfunktionen des Materials im Sensorgehäuse und des Materials der Umgebung zueinander proportional sind.

Insbesondere ist das in den Innenraum eingebrachte Material so gewählt, dass dessen Feuchtespeicherfunktion eine Zunahme der Funktionswerte der Materialfeuchte für solche Porenluftfeuchten aufweist, bei denen sich auch die Materialfeuchte des Messobjektes verändert. Durch eine Wahl des feuchtespeichernden Materials derart, dass in einem bevorzugten Messbereich kleine Änderungen der Feuchte des umgebenden Materials eine vergleichsweise große Änderung der Feuchte des feuchtespeichernden Materials im Inneren des Sensorgehäuses bewirken, kann ein besonders empfindlicher und genauer Feuchtesensor bereitgestellt werden.

Bevorzugt ist das feuchtespeichernde Material im Inneren des Sensorgehäuses als das gleiche Material gewählt, in welches der Feuchtesensor eingebracht und dessen Feuchte vermessen werden soll (Messobjekt). Unter einem (bezüglich der Feuchtespeicherung) gleichen Material soll hier und im Folgenden beispielsweise im Falle eines Estrichs, Mörtels oder Betons ein Baustoff mit derselben Rezeptur bezeichnet werden (das heißt: eine unter Verwendung gleicher Produkte und im gleichen Mischungsverhältnis von Bindemittel, Zusatzmittel/Additiven beziehungsweise Compound, Zuschlagstoffen und Zugabewasser hergestellte Stoffmischung), bei dem sich aber die Korngröße der verwendeten Zuschlagstoffe unterscheiden kann. Die Korngröße hat keinen nennenswerten Einfluss auf die Feuchtespeicherfunktion.

Bei einer solchen Ausführungsform ist der Feuchtegehalt im Material innerhalb des Sensorgehäuses gleich oder nahezu gleich zum Feuchtegehalt des Materials in dessen Umgebung, wobei ein hinreichender Feuchteausgleich zwischen Sensor und Umgebung vorausgesetzt wird. Diese Voraussetzung wurde insbesondere bei der Vermessung der Dynamik der Feuchtetransportvorgänge für einen austrocknenden Estrich experimentell und durch numerische Simulation nachgewiesen.

Mit dieser Ausführungsform ist eine besonders einfache Bestimmung des Umgebungsfeuchtegehaltes durch Messung der Resonanzfrequenz des Sensorgehäuses möglich.

Gemäß dem zweiten Aspekt der Erfindung wird bei einem Verfahren zur Feuchtemessung mittels eines Feuchtesensors nach dem ersten Aspekt der Erfindung in einem Kalibrationsschritt eine Kalibrationskurve erfasst, welche mindestens einem Wellenleitungsparameter einen Wert des Feuchtegehalts des in dem Sensorgehäuse eingebrachten feuchtespeichernden Materials zuordnet.

Wie nachfolgend anhand von Ausführungsformen noch genauer erläutert wird, kann ein Wellenleitungsparameter beispielsweise als eine Resonanzfrequenz, eine Phasendifferenz (zwischen ein- und austretendem Mikrowellensignal), eine Amplitudendifferenz (zwischen ein- und austretendem Mikrowellensignal) oder als eine Laufzeit (eine Verzögerung zwischen ein- und austretendem Mikrowellensignal) gewählt sein, wobei ein austretendes Mikrowellensignal ein an dem Feuchtesensor reflektiertes Mikrowellensignal oder ein den Feuchtesensor durchlaufendes Mikrowellensignal sein kann.

In mindestens einem nachfolgenden Messschritt wird/werden der Wert / die Werte des mindestens einen Wellenleitungsparameters des Sensorgehäuses bestimmt, welche durch die Feuchte in dessen Innenraum beeinflusst wird/werden, die sich durch den Feuchteausgleich mit der Umgebung des Sensorgehäuses einstellt.

Durch Anwendung des in der Kalibrationskurve ermittelten Zusammenhangs zwischen dem feuchteabhängigen Wert des Wellenleitungsparameters und dem Wert des Feuchtegehalts des eingebrachten Materials wird ein Wert des Feuchtegehalts des Materials der Umgebung des Sensorgehäuses ermittelt.

Ein Vorteil dieses Verfahrens besteht darin, dass auch ohne Kenntnis eines analytischen (das heißt: mathematisch exakt formulierten) Zusammenhangs zwischen dem/den Wellenleitungsparameter(n) des Sensorgehäuses und der darin angeordneten Streifenleitung und der Umgebungsfeuchte eine genaue Bestimmung der letzteren möglich ist. Weitere Vorteile des Verfahrens entsprechen den Vorteilen des Feuchtesensors gemäß dem ersten Aspekt der Erfindung.

Bei einer Ausführungsform des Verfahrens ist mindestens ein Wellenleitungsparameter als Resonanzfrequenz ausgewählt. Im Kalibrationsschritt wird eine Kalibrationskurve erfasst, welche der Resonanzfrequenz einen Wert des Feuchtegehalts des in dem Sensorgehäuse eingebrachten feuchtespeichernden Materials zuordnet. Im Messschritt wird der Wert der Resonanzfrequenz des Sensorgehäuses bestimmt, der durch die Feuchte in dessen Innenraum beeinflusst wird, die sich durch den Feuchteausgleich mit der Umgebung des Sensorgehäuses einstellt. Diese Ausführungsform hat den Vorteil, dass eine Resonanzfrequenz leicht gemessen und in einer Kalibrationskurve erfasst und ausgewertet werden kann.

Bei einer Ausführungsform wird die Resonanzfrequenz mittels eines Netzwerkanalysators als diejenige Frequenz ermittelt, bei der ein von dem Feuchtesensor reflektiertes Mikrowellensignal eine bezogen auf die Amplitude eines in den Feuchtesensor eingespeisten Mikrowellensignals minimale Amplitude aufweist. Alternativ oder zusätzlich kann bei Verwendung eines mit einem ersten und einem zweiten Koaxialkabel versehenen Feuchtesensors die Resonanzfrequenz mittels eines Netzwerkanalysators als diejenige Frequenz ermittelt werden, bei der ein von dem Feuchtesensor transmittiertes Mikrowellensignal eine bezogen auf die Amplitude eines in den Feuchtesensor eingespeisten Mikrowellensignals maximale Amplitude aufweist.

Netzwerkanalysatoren sind vergleichsweise kostengünstig und ermöglichen dadurch eine kostengünstige Messung. Zudem sind Netzwerkanalysatoren in einer mobilen Bauweise erhältlich. Somit kann insbesondere dann, wenn eine Feuchtemessung nicht fortlaufend, sondern nur zu gewissen Zeitpunkten vorgenommen werden soll, mit einem einzigen Netzwerkanalysator eine Vielzahl von Messpunkten mit je einem ortsfest verbauten Feuchtesensor erfasst und ausgewertet werden. Dies ermöglicht eine besonders kostensparende Messung.

Alternativ zu einer Messung der Resonanzfrequenz mit einem Netzwerkanalysator wird bei einem Verfahren zur Feuchtemessung mit einem Feuchtesensor gemäß dem ersten Aspekt der Erfindung mittels einer Hochfrequenzschaltung die Phasendifferenz und/oder das Amplitudenverhältnis zwischen einem von dem Feuchtesensor reflektierten und/oder (sofern dieser mit einem ersten und mit einem zweiten Koaxialkabel versehen ist) einem transmittierten Mikrowellensignal einerseits und einem in den Feuchtesensor eingespeisten Mikrowellensignal andererseits ermittelt.

Mit anderen Worten: es wird / werden mittels der Hochfrequenzschaltung die Phasendifferenz und/oder das Amplitudenverhältnis zwischen dem über ein erstes Koaxialkabel eingespeisten Mikrowellensignal einerseits und dem (bei reflektiver Messung) auf demselben Koaxialkabel reflektierten Mikrowellensignal beziehungsweise dem (bei transmissiver Messung) auf dem zweiten Koaxialkabel austretenden Mikrowellensignal ermittelt. Auch eine Kombination einer reflektiven Messung mit einer transmissiven Messung der Phasendifferenz und/oder des Amplitudenverhältnis ist möglich.

Hochfrequenzschaltungen zur Bestimmung der Phasendifferenz und des Amplitudenverhältnis von Mikrowellensignalen sind aus dem Stand der Technik bekannt.

Diesem Verfahren liegt die Erkenntnis zugrunde, dass beide Größen (das heißt: die Phasendifferenz und das Amplitudenverhältnis) mit dem Feuchtegehalt des feuchtespeichernden Materials im Sensor korreliert sind.

Mit anderen Worten: es wird die feuchteabhängige Mikrowellenleitung in dem Feuchtesensor durch eine Phasendifferenz und/oder durch eine Amplitudenänderung charakterisiert, die das Mikrowellensignal bei einer Transmission und/oder bei einer Reflexion entlang des Streifenleiters in dem Feuchtesensor erfährt. Ein Vorteil dieses Verfahrens besteht darin, dass eine Phasendifferenz und/oder eine Amplitudenänderung im Zeitbereich und somit unabhängig von einer speziellen Resonanzfrequenz ermittelt werden kann/können. Dadurch ist dieses Verfahren besonders flexibel und einfach umsetzbar.

Ferner kann bei einem Verfahren zur Feuchtemessung mit einem Feuchtesensor gemäß dem ersten Aspekt der Erfindung mittels einer Hochfrequenzschaltung auch die Laufzeit eines mittels des Feuchtesensors reflektierten und/oder transmittierten Mikrowellensignals ausgewertet werden.

Mit anderen Worten: Es wird die feuchteabhängige Mikrowellenleitung in dem Feuchtesensor durch eine Verzögerung (Laufzeit) charakterisiert, die das Mikrowellensignal bei einer Transmission und/oder bei einer Reflexion entlang des Streifenleiters in dem Feuchtesensor erfährt. Ein Vorteil dieses Verfahrens besteht darin, dass diese Laufzeit für verschiedene, unabhängig von einer Resonanzfrequenz wählbare Frequenzen oder auch für andere Signalformen (beispielsweise für impulsförmige oder stoßförmige Signalformen) ermittelt werden kann. Dadurch ist dieses Verfahren besonders flexibel und einfach umsetzbar.

Hochfrequenzschaltungen zur Bestimmung der Laufzeit von Mikrowellensignalen sind aus dem Stand der Technik bekannt.

Nach einem dritten Aspekt der Erfindung wird bei einem Verfahren zur Herstellung eines Bauteils ein Feuchtesensor gemäß dem ersten Aspekt der Erfindung in das Bauteil oder in eine Form zur Herstellung des Bauteils eingebracht und von einem Material umgeben, bevorzugt von einem fließfähigen Material umgossen. Danach wird die Feuchte in dem Bauteil nach einem Verfahren gemäß dem zweiten Aspekt der Erfindung bestimmt.

Dadurch kann, beispielsweise bei einem Beton-Fertigteil, einem Ortbeton oder einem Estrich das Fortschreiten eines Feuchtigkeit reduzierenden Vorgangs bei der Herstellung, beispielsweise eines Trocknungs- oder Abbindeprozesses, verfolgt und der Zeitpunkt, zu dem das Bauteil eingesetzt oder in weiteren Verarbeitungsschritten fertiggestellt werden kann, besonders einfach und sicher bestimmt werden. Alternativ oder zusätzlich kann auch bei einem im Einsatz befindlichen Bauteil, welches einer Feuchtigkeitsbelastung ausgesetzt ist, dessen Feuchtezustand fortlaufend ermittelt werden. Ebenfalls kann der Sensor eingesetzt werden, um eine fortschreitende Mauerwerkstrocknung nach einer Sanierungsmaßnahme (Mauerwerkstrockenlegung) nachzuweisen. Weitere Vorteile entsprechen den Vorteilen des Feuchtesensors nach dem ersten Aspekt der Erfindung und des Feuchtemessverfahrens nach dem zweiten Aspekt der Erfindung.

Bei einer Ausführungsform des Verfahrens zur Herstellung eines Bauteils wird das Bauteil aus einem fließfähigen Material hergestellt, wobei in das Sensorgehäuse dasselbe Material als feuchtespeicherndes Material eingebracht wird, in welches der Feuchtesensor in dem Bauteil eingebracht und eingebettet wird. Das Material kann in fließfähigem oder in ausgehärtetem (nicht fließfähigem) Zustand in das Sensorgehäuse eingebracht werden. Beispielsweise kann das Material vor dem Einbringen in das Sensorgehäuse als Körper (Quader) aus dem fließfähigen Material gegossen werden, in welches der Feuchtesensor eingebracht wird. Der ausgehärtete Körper (Quader) wird Festkörper in den Innenraum des Sensorgehäuses eingebracht. Alternativ oder zusätzlich wird das Sensorgehäuse mindestens teilweise mithilfe dieses Festkörpers aufgebaut. Durch das im Inneren des Sensorgehäuses gleiche Material und mittels des durch die Öffnungen im Sensorgehäuse vermittelten hygrischen Kontakts wird erreicht, dass die im Sensorgehäuse bestimmte Feuchte des feuchtespeichernden Materials gleich der Feuchte des fließfähigen Materials ist, aus dem das Bauteil hergestellt wird. Dadurch kann die Feuchte im Inneren des Bauteils besonders einfach und besonders genau bestimmt werden.

Bei einer Ausführungsform ist das fließfähige Material ein Estrichmaterial, ein Mörtel (z.B. für den Fall einer Mauerwerkssanierung), ein Fliesenkleber, Beton oder ein anderes fließfähiges Material zur Herstellung von Bauwerks-Bauteilen oder Fugen. Derartige Bauwerks-Bauteile können beispielsweise als Fertigbetonteile, aber auch als Bauteile ausgebildet sein, die aus Ortbeton oder in ähnlicher Weise unmittelbar in ein Bauwerk integriert gefertigt werden.

Die nach dieser Ausführungsform mit einem Feuchtesensor versehenen Bauteile können besonders gut in ihrer Fertigstellung, beispielsweise bei der Aushärtung eines Fliesenklebers oder bei der Hydratation eines aus Beton gefertigten Bauteils, überwacht werden. Beispielsweise kann der Zeitpunkt einer Belastbarkeit oder der Zeitpunkt, ab dem Folgearbeitsschritte begonnen werden können, besonders genau und zuverlässig bestimmt werden. Darüber hinaus eignen sich nach dieser Ausführungsform des Verfahrens hergestellte Bauteile für einen Einsatz in feuchtigkeitsexponierten Einbausituationen, indem sie mittels des eingearbeiteten Feuchtesensors fortlaufend auf einen Feuchtigkeitseintrag überwacht werden können.

Ausführungsbeispiele der Erfindung werden im Folgenden anhand von Zeichnungen näher erläutert. Darin zeigen:
- Figur 1: schematisch ein resonantes Sensorgehäuse mit zweiseitig angeschlossenen Koaxialkabeln,
- Figur 2: schematisch ein resonantes Sensorgehäuse mit einem einseitig angeschlossenen Koaxialkabel und
- Figur 3: schematisch den frequenzabhängigen reflektierten Amplitudenverlauf für in ein resonantes Sensorgehäuse eingekoppelte Mikrowellen.
Einander entsprechende Teile sind in allen Figuren mit den gleichen Bezugszeichen versehen.

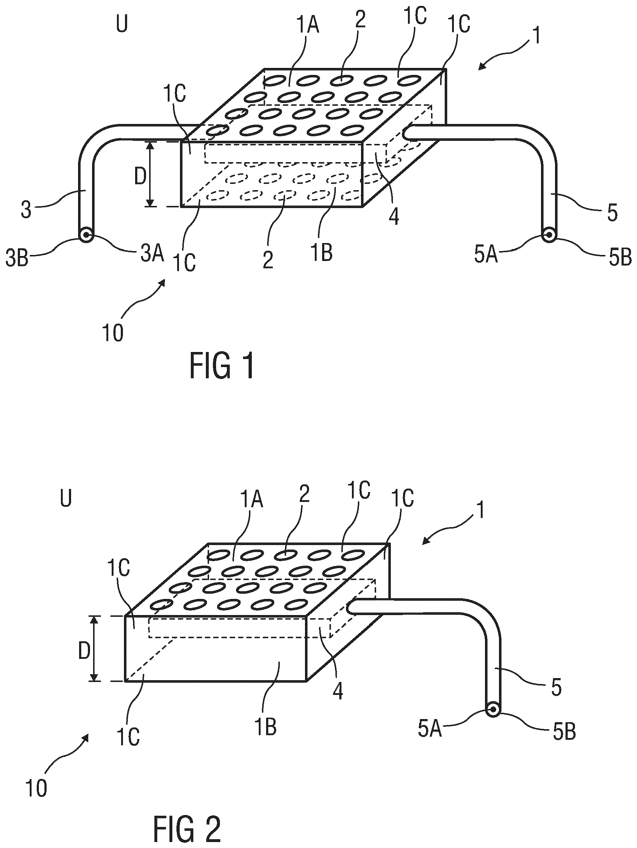

**Figur 1** zeigt schematisch einen Feuchtesensor 10 mit einem quaderförmigen Sensorgehäuse 1 mit einer für elektromagnetische Wellen resonanten Gehäusestruktur. Das Sensorgehäuse 1 ist durch aus metallischem, elektrisch leitfähigem Material oder aus mit einem solchen Material beschichteten Außenflächen 1A, 1B, 1C gebildet. Dadurch wird eine nahezu vollständige Abschirmung der elektromagnetischen Wellen bewirkt, die in das Innere des Sensorgehäuses 1 eingekoppelt werden.

Das quaderförmige Sensorgehäuse 1 weist eine obere Grundfläche 1A und eine dazu parallel angeordnete, gegenüberliegende untere Grundfläche 1B auf, die über Seitenflächen 1C miteinander elektrisch leitend verbunden sind. Die Grundflächen 1A, 1B sind in einem Abstand D beabstandet, der gering gegenüber jeder der beiden Längsausdehnungen der Grundflächen 1A, 1B ist. Beispielsweise ist der Abstand D kleiner als ein Zehntel der kleinsten Längsausdehnung der Grundflächen 1A, 1B.

In die Grundflächen 1A, 1B und optional auch in die Seitenflächen 1C sind Öffnungen 2 eingearbeitet, die für Feuchtigkeit durchlässig sind und durch die Wasser oder Wasserdampf zwischen dem Inneren des Sensorgehäuses 1 und dessen Umgebung U übertreten kann. Vorliegend sind die Öffnungen 2 kreisförmig, jedoch sind auch andere geometrische Formen möglich.

Die Ausdehnung (vorliegend der Durchmesser) einer jeden Öffnung 2 ist dabei klein gegenüber der Ausdehnung des Sensorgehäuses 1 und zudem auch klein gegenüber der Wellenlänge der elektromagnetischen Wellen, für die eine Einkopplung in das Sensorgehäuse 1 vorgesehen ist, so dass elektromagnetische Wellen nicht oder nur in sehr geringem Umfang aus dem Inneren des Sensorgehäuses 1 in die Umgebung U austreten.

Insbesondere kann ein derartiges Sensorgehäuse 1 in guter Näherung als ein Rechteckhohlleiter angesehen werden, wenn die Ausdehnung einer jeden Öffnung 2 klein gegenüber der Wellenlänge von in das Sensorgehäuse 1 eingekoppelten elektromagnetischen Wellen ist. Dadurch wird erreicht, dass das Sensorgehäuse 1 elektromagnetische Wellen mit (relativ zur Ausdehnung der Öffnungen 2) ausreichend großer Wellenlänge praktisch vollständig gegenüber der Umgebung U abschirmt.

Beispielsweise können, wenn die zur Einkopplung vorgesehenen elektromagnetischen Wellen Mikrowellen mit einer Frequenz f von zwischen 1500 Megahertz und 3000 Megahertz sind, die Öffnungen 2 als kreisförmige Öffnungen mit einem Durchmesser von zwischen einem und zwei Millimetern ausgebildet sein.

Zur Übertragung der elektromagnetischen Wellen in das / aus dem Sensorgehäuse 1 ist ein erstes Koaxialkabel 3 mit einer im Inneren des Sensorgehäuses 1 angeordneten Streifenleitung 4 verbunden. Die Streifenleitung 4 ist vorliegend als Kupferplatte oder kupferbeschichtete Platte ausgeführt und koplanar sowie näherungsweise mittig zwischen den Grundflächen 1A, 1B angeordnet. Alternativ kann die Streifenleitung 4 auch als Zwei-, Drei- oder Mehrstreifenleitung ausgeführt sein, beispielsweise als entsprechend abgeätzte kupferbeschichtete Leiterplatte.

Die Streifenleitung 4 ist elektrisch mit dem Innenleiter 3A des ersten Koaxialkabels 3 verbunden. Der Außenleiter 3B des ersten Koaxialkabels 3 ist mit den Außenflächen 1A, 1B, 1C des Sensorgehäuses 1 elektrisch verbunden. Der Eintritt des Innenleiters 3A erfolgt bevorzugt über eine der Seitenflächen 1C des Sensorgehäuses 1.

Analog sind die Streifenleitung 4 und die Außenflächen 1A, 1B, 1C mit einem zweiten Koaxialkabel 5 verbunden. Der Eintritt des Innenleiters 5A des zweiten Koaxialkabels 5 erfolgt bevorzugt durch diejenige Seitenfläche 1C hindurch, die der Seitenfläche 1C gegenüber liegt, an welcher der Innenleiter 3A des ersten Koaxialkabels 3 durchgeführt ist. Der Außenleiter 5B des zweiten Koaxialkabels 5 ist mit den Außenflächen 1A, 1B, 1C des Sensorgehäuses 1 elektrisch verbunden.

An einem derart als Hohlraumresonator ausgebildeten Sensorgehäuse 1 können alle vier komplexwertigen Streuparameter (Eingangsreflexionsfaktor S11, Rückwärts-Transmissionsfaktor S12, Vorwärts-Transmissionsfaktor S21 und Ausgangsreflexionsfaktor S22) gemessen werden. Während S11 und S22 die reflektierten Signale für das erste Koaxialkabel 3 beziehungsweise das zweite Koaxialkabel 5 beschreiben, stellt S12 das vom ersten Koaxialkabel 3 auf das zweite Koaxialkabel 5 transmittierte Signal und S21 vom zweiten Koaxialkabel 5 auf das erste Koaxialkabel 3 das transmittierte Signal dar.

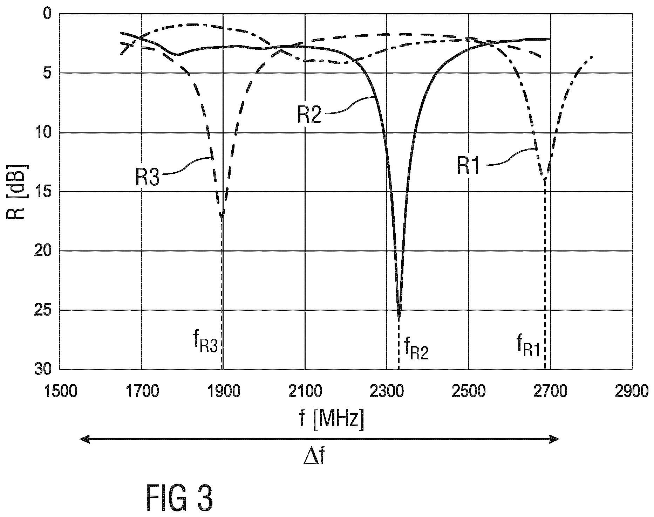

Durch die einander gegenüberliegenden, metallisch ausgebildeten und im Wesentlichen elektrisch flächig leitenden Seitenflächen 1C mit den Koaxialkabeln 3, 5 ist ein Hohlraumresonator gebildet, dessen Resonanzfrequenz *f*_{*R*1}, *f*_{*R*2}, *f*_{*R*3} durch die Geometrie des Sensorgehäuses 1 und durch das Material im Inneren des Sensorgehäuses 1, insbesondere durch dessen elektrische Permittivität bestimmt ist, wie nachfolgend anhand von Figur 3 noch genauer erläutert werden wird.

**Figur 2** zeigt eine weitere Ausführungsform für ein resonantes Sensorgehäuse 1, bei der abweichend von der in Figur 1 dargestellten Ausführungsform nur eine (vorliegend die obere) Grundfläche 1A perforiert ausgebildet, das heißt: mit Öffnungen 2 versehen ist, während die gegenüber liegende andere (vorliegend die untere) Grundfläche 1B durchgängig metallisch oder metallisch beschichtet ausgebildet ist. Ferner weist die in Figur 2 dargestellte Ausführungsform nur ein zweites Koaxialkabel 5 auf, während das in Figur 2 gegenüberliegend angeschlossene erste Koaxialkabel 3 hier weggelassen ist. Über das verbliebene zweite Koaxialkabel 5 werden sowohl die elektromagnetischen Wellen eingekoppelt als auch die reflektierten elektromagnetischen Wellen gemessen.

Diese Ausführungsform ist einfacher herstellbar und montierbar, da nur ein einzelnes (vorliegend: zweites) Koaxialkabel 5 verlegt und mit dem Sensorgehäuse 1 verbunden werden muss. Für diese Ausführungsform mit nur einem Koaxialkabel 5 lässt sich nur der Streuparameter S11 (Eingangsreflexionsfaktor) bestimmen, mit dem die vorliegende Aufgabenstellung aber grundsätzlich lösbar ist.

Der Raum über der Streifenleitung 4 im Inneren des Sensorgehäuses 1 umgebende Innenraum kann entweder zunächst leer oder aber mit Material ausgefüllt sein, das zur Aufnahme und/oder Speicherung von Feuchtigkeit geeignet ist. Wenn der Innenraum zunächst als ein leerer Raum hergestellt wird, dann kann beim Einbetten des Sensorgehäuses 1 in eine Umgebung U mit ausreichend fließfähigem Material, beispielsweise Estrichmaterial, Mörtel, Fliesenkleber, Beton, Material zum Verfüllen oder Ausgleichen von Fußböden oder ähnliches Material, dieses Material über die Öffnungen 2 in das Gehäuseinnere des Sensorgehäuses 1 eindringen, was eine vorgesehene Ausführungsvariante darstellt. Alternativ kann das Sensorgehäuse 1 auch vor dem Einbetten mit diesem Material der Umgebung U befüllt werden.

In einer besonders vorteilhaften Ausführungsform ist alles Material unterhalb der Streifenleitung 4 bis zur unteren Grundfläche 1B aus Leiterplattenmaterial gebildet, das eine möglichst kleine Feuchteaufnahmefähigkeit aufweist. Mit anderen Worten: eine auf ihrer Oberseite mit einer Streifenleitung 4 versehene, in Figur 2 nicht näher bezeichnete Leiterplatte ist mit der gegenüberliegenden Unterseite direkt auf der Innenseite der unteren Grundfläche 1B angeordnet, beispielsweise verklebt und/oder verlötet.

Der freie Raum zwischen der Oberseite der Leiterplatte und der ihr gegenüber liegenden Innenseite der oberen Grundfläche kann leer oder mit feuchtespeicherndem Material gefüllt sein. In diesen Raum eintretende Feuchte beeinflusst die Wellenleitung in dem Feuchtesensor 10. In dem Falle, dass der freie Raum oberhalb der Leiterplatte zunächst leer ist, kann er dafür vorgesehen werden, dass er ein ähnliches oder bevorzugt gleiches Material (z.B. Estrich, Mörtel, Beton) aufnimmt, wie es in der Umgebung des Sensors vorhanden ist.

Bevorzugt ist eine Streifenleitung 4 auf einer beidseitig leitfähig beschichteten Leiterplatte aufgebracht. Die mindestens im Randbereich abgeätzte Oberseite der Leiterplatte bildet dann den Streifenleiter 4 (der mit dem Innenleiter des mindestens einen Koaxialkabels 3, 5 elektrisch verbunden ist). Alternativ kann auf der Oberseite auch eine (beispielsweise mäanderformige) Leitungsstruktur eingeätzt sein. Die Unterseite der Leiterplatte wird ein Teil des abschirmenden Sensorgehäuses, indem sie mit diesem mechanisch und elektrisch verbunden (beispielsweise verlötet, optional zusätzlich verklebt) wird.

Der metallische (oder metallisch beschichtete) obere Teil des Sensorgehäuses 1 (das heißt: der das feuchtespeichernde Material umhüllende Bereich oberhalb der Oberseite der Leiterplatte) wird mit der elektrischen Masse des mindestens einen Koaxialkabels 3, 5 (das heißt: mit dessen Außenleiter 3B, 5B) und der Unterseite der Leiterplatte (beispielsweise durch Löten) elektrisch leitfähig so verbunden, dass keine offenen Schlitze verbleiben. Dadurch wird ein mechanisch besonders fester Streifenleiter 4 gebildet, der sehr einfach montiert und justiert werden kann.

Der Erfindung liegt die Erkenntnis zu Grunde, dass die Wechselwirkung von elektromagnetischen Wellen insbesondere im Mikrowellenbereich, bevorzugt in einem Frequenzbereich zwischen 1 Gigahertz und 6 Gigahertz, wesentlich von der Feuchtigkeit im Inneren des Sensorgehäuses 1 bestimmt wird. Durch die gute elektromagnetische Abschirmung des Sensorgehäuses 1 wird dabei erreicht, dass die Wechselwirkung dieser elektromagnetischen Wellen mit der Umgebung U des Sensorgehäuses 1 demgegenüber gering ist und vernachlässigt werden kann.

Im Wesentlichen wird die Wechselwirkung von elektromagnetischer Mikrowellenstrahlung mit Feuchtigkeit im Inneren des Sensorgehäuses 1 von der ständigen Ausrichtung der Dipole im elektromagnetischen Wechselfeld bestimmt, durch die der eintretenden Mikrowellenstrahlung Energie entzogen und in Wärme umgewandelt wird. Entsprechend verringert sich die Energie der elektromagnetischen Wellen, die in dem Sensorgehäuse 1 reflektiert und über das erste Koaxialkabel 3 zurückgeleitet und/oder über das zweite Koaxialkabel 5 weitergeleitet werden.

Figuren 1 und 2 sind lediglich Ausführungsbeispiele des prinzipiellen Aufbaus eines Feuchtesensors 10. Abweichend davon ist beispielsweise auch eine Kombination des in Figur 1 dargestellten oben und unten perforierten Sensorgehäuses 1 mit nur einem der in Figur 1 dargestellten zwei Koaxialkabel 3, 5 gemäß Figur 2 möglich. Außerdem sind weitere Geometrien eines von dem Sensorgehäuse 1 gebildeten Hohlraumresonators denkbar, zum Beispiel eine Zylindergeometrie.

Beispielhaft und schematisch ist in **Figur 3** der frequenzabhängige logarithmische Dämpfungsverlauf von elektromagnetischen Wellen dargestellt, die über das erste Koaxialkabel 3 eingespeist werden, von der Streifenleitung 4 in das Gehäuseinnere des Sensorgehäuses 1 abgestrahlt werden und dort in Wechselwirkung mit Wassermolekülen treten. Weist das Sensorgehäuse 1 lediglich ein erste Koaxialkabel 3 gemäß der Ausführungsform nach Figur 2 auf, so wird der wesentliche Anteil der eingespeisten Energie, der nicht an die Wassermoleküle übertragen wird, über das erste Koaxialkabel 3 reflektiert.

Der Anteil der reflektierten Wellenenergie hängt hierbei von der Frequenz *f* der Mikrowellen ab und ist für eine Resonanzfrequenz *f*_{*R*1}, *f*_{*R*2}, *f*_{*R*3} besonders niedrig. Mit anderen Worten: mit der Resonanzfrequenz *f*_{*R*1}, *f*_{*R*2}, *f*_{*R*3} eingespeiste Mikrowellen treten in besonders hohem Maß in Wechselwirkung mit den Wassermolekülen im Gehäuseinneren und werden in entsprechend besonders geringem Maß reflektiert und/oder (sofern ein zweites Koaxialkabel 5 angeschlossen ist) weitergeleitet.

Dabei hat sich herausgestellt, dass die Resonanzfrequenz *f*_{*R*1}, *f*_{*R*2}, *f*_{*R*3} von der Feuchtigkeit im Gehäuseinneren abhängt und sich mit zunehmender Feuchtigkeit nach geringeren Frequenzen *f* hin verschiebt. Das Material und dessen Feuchtigkeit in der Umgebung U des Sensorgehäuses 1 hat durch die nahezu vollständige Abschirmung der elektromagnetischen Wellen durch das Sensorgehäuse 1 keine oder nur eine vernachlässigbar kleine direkte Auswirkung auf die Lage der Resonanzfrequenz *f*_{*R*1}, *f*_{*R*2}, *f*_{*R*3}. Ebenso wird die Resonanzfrequenz *f*_{*R*1}, *f*_{*R*2}, *f*_{*R*3} durch Strukturen (beispielsweise metallische Objekte oder wechselnde Materialschichten) in der Umgebung U nicht beeinflusst. Selbstverständlich beeinflusst die Feuchtigkeit der Umgebung U aber durch Diffusionsaustausch die Feuchtigkeit im Inneren des Sensorgehäuses 1 und damit indirekt die Lage der Resonanzfrequenz *f*_{*R*1}, *f*_{*R*2}, *f*_{*R*3}.

In Figur 3 sind zur Verdeutlichung ein erster bis dritter frequenzabhängiger Reflexionsverlauf R1 bis R3 eines Reflexionsgrads R, angegeben als logarithmischer Reflexionsgrad in Dezibel (dB), dargestellt. Jedem Reflexionsverlauf R1 bis R3 ist jeweils eine Resonanzfrequenz *f*_{*R*1}, *f*_{*R*2}, *f*_{*R*3} als diejenige Frequenz *f* zugeordnet, bei der der Reflexionsverlauf R1 bis R3 sein jeweiliges Minimum erreicht. Der erste Reflexionsverlauf R1 mit der ersten Resonanzfrequenz *f*_{*R*1} ist einer geringen Feuchtigkeit (im Inneren des Sensorgehäuses 1) zugeordnet. Der zweite Reflexionsverlauf R2 mit der zweiten Resonanzfrequenz *f*_{*R*2} ist einer mittleren Feuchtigkeit und der dritte Reflexionsverlauf R3 mit der dritten Resonanzfrequenz *f*_{*R*3} ist einer hohen Feuchtigkeit (bei ansonsten unverändertem Material im Gehäuseinneren) zugeordnet.

Die Lage der Resonanzfrequenz *f*_{*R*1}, *f*_{*R*2}, *f*_{*R*3} kann durch Vermessung des (über das erste Koaxialkabel 3) reflektierten Signals und/oder durch Vermessung des (über das gegebenenfalls angeordnete zweite Koaxialkabel 5) weitergeleiteten Signals beispielsweise mittels eines Netzwerkanalysators bestimmt werden. Preiswerte Netzwerkanalysatoren, mit denen ein frequenzabhängiger Reflexionsverlauf R1 bis R3 über ein Frequenzband Δ*f* hinweg bestimmt werden kann, sind aus dem Stand der Technik bekannt, beispielsweise das Produkt LibreVNA als ein Open-Source-Vektor-Netzwerkanalysator. Hierbei muss das Frequenzband Δ*f* so gewählt werden, dass es für jede zu erwartende Feuchtigkeit die zugeordnete Resonanzfrequenz *f*_{*R*1}, *f*_{*R*2}, *f*_{*R*3} überdeckt.

Die Lage der Resonanzfrequenz *f*_{*R*1}, *f*_{*R*2}, *f*_{*R*3} kann alternativ oder zusätzlich auch durch Messverfahren ermittelt werden, bei denen nicht nur die Amplitude des reflektierten und/oder weitergeleiteten Mikrowellensignals, sondern auch dessen Phasenlage (bezogen auf die Phasenlage des über das erste Koaxialkabel 3 eingespeisten Mikrowellensignals) ausgewertet wird. Beispielsweise kann aus der Phasenverschiebung (das heißt: dem Versatz der Phasenlagen des eingespeisten Mikrowellensignals einerseits und des reflektierten oder weitergeleiteten Mikrowellensignals andererseits) bei einer gewissen, vorbestimmten Frequenz *f*, beispielsweise bei 300 Megahertz, die Lage der Resonanzfrequenz *f*_{*R*1}, *f*_{*R*2}, *f*_{*R*3} bestimmt werden.

Bei diesem Ansatz ist es nicht erforderlich, den Reflexionsgrad R über das Frequenzband Δ*f* hinweg zu ermitteln, so dass zwar eine Hochfrequenzschaltung zur Ermittlung der Phasendifferenz zwischen eingekoppeltem und reflektiertem Mikrowellensignal, nicht jedoch ein kompletter Netzwerkanalysator erforderlich ist. Dadurch kann mit einer besonders einfachen und kostensparenden Messvorrichtung die Feuchtigkeit der Umgebung U ermittelt werden.

Für die Zuordnung eines Feuchtewertes zu einer (beispielsweise mittels Netzwerkanalysator oder alternativ mittels Hochfrequenzschaltung bestimmten) Resonanzfrequenz *f*_{*R*1}, *f*_{*R*2}, *f*_{*R*3} wird sich eine Kalibration als hilfreich erweisen. Bei einer Kalibration wird für ein gewisses Material im Gehäuseinneren des Sensorgehäuses 1 dessen Feuchtewert variiert und die vom variablen Feuchtewert abhängige Lage der Resonanzfrequenz *f*_{*R*1}, *f*_{*R*2}, *f*_{*R*3} ermittelt. Das Ergebnis der Kalibration kann als Kalibrationskurve dargestellt werden, mit der jeder nachfolgend ermittelten Resonanzfrequenz *f*_{*R*1}, *f*_{*R*2}, *f*_{*R*3} für das betreffende Material im Gehäuseinneren ein Feuchtewert zugeordnet wird.

Wenn das Material im Gehäuseinneren des Sensorgehäuses 1 dasselbe Material wie in der zu vermessenden Umgebung U ist, so kann mittels dieser Kalibrationskurve der Feuchtewert der Umgebung U unmittelbar bestimmt werden, wenn durch die Öffnungen 2 im Sensorgehäuse 1 ein guter hygrischer Kontakt zwischen dem Gehäuseinneren und der Umgebung U hergestellt ist, beispielsweise indem das Sensorgehäuse 1 bei der Herstellung eines Bauteils aus einem fließfähigen Material in dieses Bauteil mit eingegossen wird. Durch den guten hygrischen Kontakt gleicht sich die Feuchtigkeit zwischen dem Inneren des Sensorgehäuses 1 und dem Bauteil in dessen Umgebung U aus.

Da die Feuchtespeichereigenschaften im Gehäuseinneren und in der Umgebung U (wegen des gleichen Materials) gleich sind, das heißt: da sich bei gleicher Porenluftfeuchte oder bei gleichem Kapillardruck auch dieselbe Materialfeuchte einstellt, entspricht der anhand der gemessenen Resonanzfrequenz *f*_{*R*1}, *f*_{*R*2}, *f*_{*R*3} durch Anwendung der Kalibrationskurve ermittelte Feuchtewert im Gehäuseinneren dem Feuchtewert im Material des Bauteils, das das Sensorgehäuse 1 umgibt.

Wenn im Gehäuseinneren des Sensorgehäuses 1 ein Material mit bekannten Feuchtespeichereigenschaften eingebracht ist, das sich von dem Material in der Umgebung U (also beispielsweise in dem Bauteil, in welches das Sensorgehäuse 1 eingegossen wurde) unterscheidet, so kann durch Messung der Resonanzfrequenz *f*_{*R*1}, *f*_{*R*2}, *f*_{*R*3} das Feuchtepotential (das heißt: der Kapillardruck oder die Porenluftfeuchte) bestimmt werden. Nach aus dem Stand der Technik bekannten Verfahren der Ausgleichsfeuchtemessung kann aus diesem Kapillardruck oder aus dieser Porenluftfeuchte durch Anwendung der Feuchtespeicherfunktion des Materials in der Umgebung U des Sensorgehäuses 1 ebenfalls dessen Materialfeuchte bestimmt werden.

Das elektromagnetisch schirmend ausgebildete Sensorgehäuse 1 bewirkt, dass eingekoppelte elektromagnetische Felder, insbesondere Mikrowellen, nicht oder nur vernachlässigbar aus dem Sensorgehäuse 1 abgestrahlt werden. Die Lage der Resonanzfrequenz *f*_{*R*1}, *f*_{*R*2}, *f*_{*R*3} ist damit durch den Feuchtegehalt des feuchtespeichernden Materials im Inneren des Sensorgehäuses 1 bestimmt. Die Resonanzfrequenz *f*_{*R*1}, *f*_{*R*2}, *f*_{*R*3} wird sich in Verbindung mit der hygrischen Ankopplung des Messobjektes in der Umgebung U nicht verändern. Auch haben die dielektrischen Eigenschaften des Materials in der Umgebung U und die geometrischen Abmessungen eines solchen Materials keinen Einfluss auf die anhand beispielsweise des reflektierten oder transmittierten Mikrowellensignals ermittelte Resonanzfrequenz *f*_{*R*1}, *f*_{*R*2}, *f*_{*R*3}*.* Eine Wechselwirkung zwischen dem Inneren des Sensorgehäuses 1 und dessen Umgebung U erfolgt nur durch Feuchteaustausch über die Öffnungen 2.

In einer weiteren Anwendung kann mit dem Sensorgehäuse 1 auch eine Luftfeuchte ermittelt werden, insbesondere in Umgebungen U mit sehr hoher Luftfeuchte, beispielsweise mit einer relativen Luftfeuchte von über 95 Prozent. Hierbei wird es sich als hilfreich erweisen, in das Innere des Sensorgehäuses 1 ein Material einzubringen, das für sehr hohe Luftfeuchtewerte eine hohe Feuchtespeicherung aufweist.

### BEZUGSZEICHENLISTE

- 1: Sensorgehäuse
- 1A: obere Grundfläche, Außenfläche
- 1B: untere Grundfläche, Außenfläche
- 1C: Seitenfläche, Außenfläche
- 2: Öffnung
- 3, 5: erstes, zweites Koaxialkabel
- 3A, 5A: Innenleiter
- 3B, 5B: Außenleiter
- 4: Streifenleitung
- 10: Feuchtesensor

- D: Abstand
- *f*: Frequenz
- *f*_{*R*1}, *f*_{*R*2}, *f*_{*R*3}: erste bis dritte Resonanzfrequenz
- Δ*f*: Frequenzband
- R: Reflexionsgrad
- R1, R2, R3: erster bis dritter Reflexionsverlauf
- U: Umgebung

## Patentansprüche

1. Feuchtesensor (10) umfassend ein elektromagnetisch abschirmendes Sensorgehäuse (1) und einen darin angeordneten Streifenleiter (4) sowie mindestens ein Koaxialkabel (3, 5) mit je einem mit dem Streifenleiter (4) elektrisch verbundenen Innenleiter (3A, 5A) und einem mit dem Sensorgehäuse (1) elektrisch verbundenen Außenleiter (3B, 5B), wobei der Streifenleiter (4) und das mindestens eine Koaxialkabel (3, 5) zur Leitung von Mikrowellen mit einer Frequenz (*f*) in einem Frequenzband (Δ*f*) eingerichtet sind, welches mindestens einen Teilbereich des Mikrowellenbereichs von zwischen 10 Megahertz (MHz) und 300 GHz überdeckt, wobei das Sensorgehäuse (1) als Hohlraumresonator mit einer Resonanzfrequenz (*f*_{*R*1}, *f*_{*R*2}, *f*_{*R*3}) innerhalb des Frequenzbands (Δ*f*) und für das Frequenzband (Δ*f*) abschirmend ausgebildet ist und Öffnungen (2) aufweist, die einen für einen Feuchteausgleich ausreichenden hygrischen Kontakt zwischen dem Inneren und der Umgebung (U) des Sensorgehäuses (1) herstellen und wobei in den Innenraum des Sensorgehäuses (1) feuchtespeicherndes Material eingebracht ist.

2. Feuchtesensor (10) nach Anspruch 1, **dadurch gekennzeichnet, dass** ein erstes Koaxialkabel (3) in das Sensorgehäuse (1) geführt und mit seinem Innenleiter (3A) mit einem ersten Ende der Streifenleitung (4) elektrisch verbunden ist und ein zweites Koaxialkabel (5) in das Sensorgehäuse (1) geführt und mit seinem Innenleiter (5A) mit einem dem ersten Ende gegenüberliegenden zweiten Ende der Streifenleitung (4) elektrisch verbunden ist.

3. Feuchtesensor (10) nach Anspruch 1 oder 2, **dadurch gekennzeichnet, dass** das Sensorgehäuse (1) als Rechteck-Hohlleiter ausgebildet ist und zwei kongruente Grundflächen (1A, 1B) aufweist, die in einem Abstand (D) kleiner als die kleinste Abmessung der Grundflächen (1A, 1B) einander parallel gegenüberliegend angeordnet und über Seitenflächen (1C) verbunden sind, wobei in mindestens eine Grundfläche (1A, 1B) Öffnungen (2) zum Feuchteausgleich eingebracht sind und wobei mindestens ein Koaxialkabel (3, 5) durch jeweils eine der Seitenflächen (1C) in das Sensorgehäuse (1) geführt ist.

4. Feuchtesensor (10) nach einem der vorhergehenden Ansprüche, **dadurch gekennzeichnet, dass** die Streifenleitung (4) als Kupferplatte ausgeführt ist.

5. Feuchtesensor (10) nach einem der Ansprüche 1 bis 3, **dadurch gekennzeichnet, dass** die Streifenleitung (4) als kupferbeschichtete Leiterplatte mit einer Leitungsführung als Einstreifenleitung oder als Mehrstreifenleitung oder als Leiterstruktur umfassend eine Einstreifenleitung und/oder eine Mehrstreifenleitung ausgeführt ist.

6. Feuchtesensor (10) nach einem der vorhergehenden Ansprüche, **dadurch gekennzeichnet, dass** das im Innenraum des Sensorgehäuses (1) eingebrachte Material eine gleiche oder ähnliche Feuchtespeicherfunktion aufweist wie das Material in der zu vermessenden Umgebung (U).

7. Verfahren zur Feuchtemessung mit einem Feuchtesensor (10) nach einem der vorhergehenden Ansprüche, **dadurch gekennzeichnet, dass** in einem Kalibrationsschritt eine Kalibrationskurve erfasst wird, welche mindestens einem Wellenleitungsparameter einen Feuchtewert des in dem Sensorgehäuse (1) eingebrachten Materials zuordnet und in mindestens einem nachfolgenden Messschritt der Wert des mindestens einen Wellenleitungsparameters gemessen und durch Anwendung der Kalibrationskurve ein Feuchtewert der Umgebung (U) ermittelt wird.

8. Verfahren nach Anspruch 7, **dadurch gekennzeichnet, dass** mindestens ein Wellenleitungsparameter als Resonanzfrequenz (*f*_{*R*1}, *f*_{*R*2}, *f*_{*R*3}) in dem Kalibrationsschritt erfasst und in dem nachfolgenden Messschritt in seinem Wert bestimmt wird.

9. Verfahren nach Anspruch 8, **dadurch gekennzeichnet, dass** die Resonanzfrequenz (*f*_{*R*1}, *f*_{*R*2}, *f*_{*R*3}) mittels eines Netzwerkanalysators als diejenige Frequenz (*f*) ermittelt wird, bei der ein von dem Feuchtesensor (10) reflektiertes und/oder transmittiertes Mikrowellensignal eine bezogen auf die Amplitude eines in den Feuchtesensor (10) eingespeisten Mikrowellensignals extremale Amplitude aufweist.

10. Verfahren nach einem der Ansprüche 7 bis 9, **dadurch gekennzeichnet, dass** mindestens ein Wellenleitungsparameter mittels einer Hochfrequenzschaltung als die Phasendifferenz und/oder als das Amplitudenverhältnis eines von dem Feuchtesensor (10) reflektierten und/oder transmittierten Mikrowellensignal einerseits und eines in den Feuchtesensor (10) eingespeisten Mikrowellensignal andererseits ermittelt wird.

11. Verfahren nach einem der Ansprüche 7 bis 10, **dadurch gekennzeichnet, dass** mittels einer Hochfrequenzschaltung die Laufzeit eines mittels des Feuchtesensors (10) transmittierten und/oder reflektierten Mikrowellensignals ausgewertet wird.

12. Verfahren zur Herstellung eines Bauteils, wobei ein Feuchtesensor (10) nach einem der Ansprüche 1 bis 6 in das Bauteil eingebracht und von einem fließfähigen Material umgossen wird und danach die Feuchte in dem Bauteil mit einem Verfahren nach einem der Ansprüche 7 bis 11 bestimmt wird, wobei in das Sensorgehäuse (1) des Feuchtesensors (10) dasselbe Material eingebracht wird, in welches der Feuchtesensor (10) in dem Bauteil eingebracht wird.

13. Verfahren nach Anspruch 12, **dadurch gekennzeichnet, dass** das in das Sensorgehäuse eingebrachte Material fließfähig ist und als Estrichmaterial, Mörtel, Fliesenkleber, Beton oder als ein fließfähiges Material zur Herstellung von Bauwerks-Bauteilen ausgebildet ist.

## Claims

1. Moisture sensor (10) comprising an electromagnetically shielding sensor housing (1) and a strip conductor (4) arranged therein and also at least one coaxial cable (3, 5), each having an inner conductor (3A, 5A) electrically connected to the strip conductor (4) and an outer conductor (3B, 5B) electrically connected to the sensor housing (1), wherein the strip conductor (4) and the at least one coaxial cable (3, 5) are designed for conducting microwaves with a frequency (f) in a frequency band (Δ*f*) which covers at least a portion of the microwave range of between 10 megahertz (MHz) and 300 GHz, wherein the sensor housing (1) is in the form of a cavity resonator with a resonant frequency (*f*_{*R*1}, *f*_{*R*2}, *f*_{*R*3}) within the frequency band (Δ*f*) and in a shielding manner for the frequency band (Δ*f*) and has openings (2), which establish hygric contact between the interior and the area (U) surrounding the sensor housing (1) that is sufficient for moisture compensation, and wherein moisture-storing material is introduced into the interior of the sensor housing (1).

2. Moisture sensor (10) according to Claim 1, **characterized in that** a first coaxial cable (3) is fed into the sensor housing (1) and by way of its inner conductor (3A) is electrically connected to a first end of the strip conductor (4), and a second coaxial cable (5) is fed into the sensor housing (1) and by way of its inner conductor (5A) is electrically connected to a second end of the strip conductor (4), the second end being situated opposite the first end.

3. Moisture sensor (10) according to Claim 1 or 2, **characterized in that** the sensor housing (1) is in the form of a rectangular waveguide and has two congruent base surfaces (1A, 1B), which are arranged parallel and opposite each other at a distance (D) of less than the smallest dimension of the base surfaces (1A, 1B) and are connected via side surfaces (1C), wherein openings (2) are made in at least one base surface (1A, 1B) for moisture compensation and wherein at least one coaxial cable (3, 5) is led through in each case one of the side surfaces (1C) into the sensor housing (1).

4. Moisture sensor (10) according to any of the preceding claims, **characterized in that** the strip conductor (4) is embodied as a copper plate.

5. Moisture sensor (10) according to any of Claims 1 to 3, **characterized in that** the strip conductor (4) is embodied as a copper-coated printed circuit board with a line guide as a single-strip conductor or as a multi-strip conductor or as a conductor structure comprising a single-strip conductor and/or a multi-strip conductor.

6. Moisture sensor (10) according to any of the preceding claims, **characterized in that** the material introduced into the interior of the sensor housing (1) has the same or a similar moisture storage function as/to the material in the surrounding area (U) to be measured.

7. Method for measuring moisture using a moisture sensor (10) according to any of the preceding claims, **characterized in that**, in a calibration step, a calibration curve is detected, which assigns a moisture value of the material introduced in the sensor housing (1) to at least one waveguide parameter and, in at least one subsequent measurement step, the value of the at least one waveguide parameter is measured and a moisture value of the surrounding area (U) is determined by applying the calibration curve.

8. Method according to Claim 7, **characterized in that** at least one waveguide parameter is detected as the resonant frequency (*f*_{*R*1}, *f*_{*R*2}, *f*_{*R*3}) in the calibration step and its value is determined in the subsequent measurement step.

9. Method according to Claim 8, **characterized in that** the resonant frequency (*f*_{*R*1}, *f*_{*R*2}, *f*_{*R*3}) is determined by means of a network analyser as that frequency (*f*) at which a microwave signal reflected and/or transmitted by the moisture sensor (10) has an extreme amplitude based on the amplitude of a microwave signal fed into the moisture sensor (10).

10. Method according to any of Claims 7 to 9, **characterized in that** at least one waveguide parameter is determined by means of a high-frequency circuit as the phase difference and/or as the amplitude ratio of a microwave signal reflected and/or transmitted by the moisture sensor (10) on the one hand and a microwave signal fed into the moisture sensor (10) on the other.

11. Method according to any of Claims 7 to 10, **characterized in that** the propagation time of a microwave signal transmitted and/or reflected by means of the moisture sensor (10) is evaluated by means of a high-frequency circuit.

12. Method for producing a component, wherein a moisture sensor (10) according to any of Claims 1 to 6 is introduced into the component and is surrounded by a flowable material and then the moisture in the component is determined by a method according to any of Claims 7 to 11, wherein the same material into which the moisture sensor (10) is introduced into the component is introduced into the sensor housing (1) of the moisture sensor (10).

13. Method according to Claim 12, **characterized in that** the material introduced into the sensor housing is flowable and is in the form of screed material, mortar, tile adhesive, concrete or as a flowable material for producing construction components.

## Revendications

1. Capteur d'humidité (10) comprenant un boîtier de capteur (1) à blindage électromagnétique et un conducteur en bande (4) agencé à l'intérieur de celui-ci, ainsi qu'au moins un câble coaxial (3, 5) chacun avec un conducteur intérieur (3A, 5A) relié électriquement au conducteur en bande (4) et un conducteur extérieur (3B, 5B) relié électriquement au boîtier de capteur (1), le conducteur en bande (4) et ledit au moins un câble coaxial (3, 5) étant conçus pour conduire des micro-ondes d'une fréquence (*f*) située dans une bande de fréquences (Δ*f*) qui couvre au moins une partie de la gamme des micro-ondes comprise entre 10 mégahertz (MHz) et 300 gigahertz (GHz), le boîtier du capteur (1) étant conçu sous la forme d'un résonateur à cavité avec une fréquence de résonance (*f*_{*R*1}, *f*_{*R*2}, *f*_{*R*3}) située à l'intérieur de la bande de fréquence *(Δf)* et blindant pour la bande de fréquence *(Δf)* et présentant des ouvertures (2) qui établissent un contact hygrique entre l'intérieur du boîtier de capteur (1) et l'environnement (U) de ce dernier, suffisant pour une compensation de l'humidité, et dans lequel un matériau absorbant l'humidité est introduit dans l'espace intérieur du boîtier de capteur (1).

2. Capteur d'humidité (10) selon la revendication 1, **caractérisé en ce qu'**un premier câble coaxial (3) est introduit dans le boîtier du capteur (1) et son conducteur intérieur (3A) est relié électriquement à une première extrémité du conducteur en bande (4), et **en ce qu'**un deuxième câble coaxial (5) est introduit dans le boîtier du capteur (1) et son conducteur intérieur (5A) est relié électriquement à une deuxième extrémité du conducteur en bande (4) opposée à la première extrémité.

3. Capteur d'humidité (10) selon la revendication 1 ou la revendication 2, **caractérisé en ce que** le boîtier du capteur (1) est conçu sous la forme d'un guide d'ondes rectangulaire et présente deux surfaces de base congruentes (1A, 1B) qui sont agencées parallèlement l'une en face de l'autre à une distance (D) inférieure à la plus petite dimension des surfaces de base (1A, 1B), parallèles l'une à l'autre et agencées en face l'une de l'autre, et reliées par des surfaces latérales (1C), des ouvertures (2) étant ménagées dans au moins une surface de base (1A, 1B) pour compenser l'humidité, et au moins un câble coaxial (3, 5) étant introduit dans le boîtier du capteur (1) à travers l'une des surfaces latérales (1C).

4. Capteur d'humidité (10) selon l'une des revendications précédentes, **caractérisé en ce que** le conducteur en bande (4) est réalisé sous la forme d'une plaque de cuivre.

5. Capteur d'humidité (10) selon l'une des revendications 1 à 3, **caractérisé en ce que** le conducteur en bande (4) est réalisé sous la forme d'une carte à circuit imprimé revêtue de cuivre avec un tracé de ligne sous la forme d'une ligne monobande ou d'une ligne multibande ou sous la forme d'une structure conductrice comprenant une ligne monobande et/ou une ligne multibande.

6. Capteur d'humidité (10) selon l'une des revendications précédentes, **caractérisé en ce que** le matériau introduit à l'intérieur du boîtier du capteur (1) présente une fonction de stockage de l'humidité identique ou similaire à celle du matériau se trouvant dans l'environnement (U) à mesurer.

7. Procédé de mesure de l'humidité au moyen d'un capteur d'humidité (10) selon l'une des revendications précédentes, **caractérisé en ce qu'**une courbe d'étalonnage est enregistrée lors d'une étape d'étalonnage, laquelle associe une valeur d'humidité du matériau introduit dans le boîtier du capteur (1) à au moins un paramètre de guide d'ondes, et la valeur d'au moins un paramètre de guide d'ondes est mesurée lors d'au moins une étape de mesure suivante, et une valeur d'humidité de l'environnement (U) est déterminée en utilisant la courbe d'étalonnage.

8. Procédé selon la revendication 7, **caractérisé en ce qu'**au moins un paramètre de guide d'ondes est saisi en tant que fréquence de résonance (*f*_{*R*1}, *f*_{*R*2}, *f*_{*R*3}) dans l'étape d'étalonnage et sa valeur est déterminée dans l'étape de mesure suivante

9. Procédé selon la revendication 8, **caractérisé en ce que** la fréquence de résonance (*f*_{*R*1}, *f*_{*R*2}, *f*_{*R*3}) est déterminée au moyen d'un analyseur de réseau comme étant la fréquence (*f*) à laquelle un signal micro-ondes réfléchi et/ou transmis par le capteur d'humidité (10) présente une amplitude extrême par rapport à l'amplitude d'un signal micro-ondes introduit dans le capteur d'humidité (10).

10. Procédé selon l'une des revendications 7 à 9, **caractérisé en ce qu'**au moins un paramètre de guide d'ondes est déterminé au moyen d'un circuit haute fréquence en tant que différence de phase et/ou en tant que rapport d'amplitude d'un signal micro-ondes réfléchi et/ou transmis par le capteur d'humidité (10), d'une part, et d'un signal micro-ondes introduit dans le capteur d'humidité (10), d'autre part.

11. Procédé selon l'une des revendications 7 à 10, **caractérisé en ce que** le temps de propagation d'un signal micro-ondes transmis et/ou réfléchi par le capteur d'humidité (10) est évalué au moyen d'un circuit haute fréquence.

12. Procédé de fabrication d'un composant, dans lequel un capteur d'humidité (10) selon l'une des revendications 1 à 6 est inséré dans le composant et enrobé d'un matériau fluide, puis l'humidité dans le composant est déterminée par un procédé selon l'une des revendications 7 à 11, le même matériau étant introduit dans le boîtier (1) du capteur d'humidité (10) que celui dans lequel le capteur d'humidité (10) est inséré dans le composant.

13. Procédé selon la revendication 12, **caractérisé en ce que** le matériau inséré dans le boîtier du capteur est fluide et se présente sous la forme d'un matériau de chape, de mortier, de colle à carrelage, de béton ou d'un matériau fluide pour la fabrication de composants de construction.
